# EUROPEAN PATENT SPECIFICATION

(11) **EP 3 985 396 B1**
(45) Date of publication and mention of the grant of the patent: **18.11.2026**
(21) Application number: 20821764.6
(22) Date of filing: 12.06.2020
(51) Int. Cl.: G01N 33/68, A61K 31/4418, A61K 31/496, A61K 31/675, A61K 45/00, A61L 27/36, A61P 11/00, C07K 16/18, G01N 33/53

(54) **PROGNOSIS PREDICTION METHOD OF IDIOPATHIC PULMONARY FIBROSIS**
PROGNOSEPRÄDIKTIONSVERFAHREN FÜR IDIOPATHISCHE LUNGENFIBROSE
MÉTHODE DE PRÉDICTION DE PRONOSTIC DE FIBROSE PULMONAIRE IDIOPATHIQUE

(30) Priority: 14.06.2019 JP 2019111310
(43) Date of publication of application: 20.04.2022
(73) Proprietor: National University Corporation Hamamatsu University School of Medicine, Hamamatsu-shi, Shizuoka 431-3192 (JP); Medical & Biological Laboratories Co., Ltd., Tokyo, 105-0012 (JP)
(72) Inventor: HOZUMI Hironao, Hamamatsu-shi, Shizuoka 431-3192 (JP); SUDA Takafumi, Hamamatsu-shi, Shizuoka 431-3192 (JP); SUGIURA Katsunori, Ina-shi, Nagano 396-0002 (JP); MORITA Yuka, Ina-shi, Nagano 396-0002 (JP); KARASAWA Chigusa, Ina-shi, Nagano 396-0002 (JP)
(74) Representative: J A Kemp LLP
(86) International application number: PCT/JP2020/023227
(87) International publication number: WO 2020/251024

(56) References cited:
- WO-A1-2014/068300
- WO-A1-2014/144821
- WO-A2-2014/127290
- JP-A- 2008 533 155
- JP-A- 2018 083 845
- JP-A- 2018 127 443
- JP-A- H10 503 365
- LEY BRETT ET AL: "Clinical Course and Prediction of Survival in Idiopathic Pulmonary Fibrosis", vol. 183, no. 4, 15 February 2011 (2011-02-15), US, pages 431 - 440, XP055963952, ISSN: 1073-449X, Retrieved from the Internet <URL:https://www.atsjournals.org/doi/pdf/10.1164/rccm.201006-0894CI> DOI: 10.1164/rccm.201006-0894CI
- AKIYAMA ET AL: "Clinical Utility of Serum S100 Calcium Binding Protein A4 in Idiopathic Pulmonary Fibrosis", THE AMERICAN REVIEW OF RESPIRATORY DISEASE, AMERICAN THORACIC SOCIETY, US, vol. 199, no. 9, 22 May 2019 (2019-05-22), pages A7131, XP009532348, ISSN: 0003-0805
- GODWIN ANDREW ET AL: "Blocking Cold-Inducible RNA-Binding Protein Protects Liver From Ischemia-Reperfusion Injury", SHOCK, vol. 43, no. 1, 1 January 2015 (2015-01-01), US, pages 24 - 30, XP093034938, ISSN: 1073-2322, DOI: 10.1097/SHK.0000000000000251
- AKIYAMA, N. ET AL.: "Clinical Utility of Serum S100 Calcium Binding Protein A4 in Idiopathic Pulmonary Fibrosis", AMERICAN JOURNAL OF RESPIRATORY AND CRITICAL CARE MEDICINE, vol. 199, no. 9, 22 May 2019 (2019-05-22), US , pages A7131, XP009532348, ISSN: 0003-0805
- TANAKA, KAZUKI ET AL.: "Examination of the usefulness of NGAL, S100-A9, S100-A8 measurement in acute exacerbation of idiopathic pulmonary fibrosis,", ANNALS OF THE JAPANESE RESPIRATORY SOCIETY, vol. 8, 28 February 2019 (2019-02-28), pages 255, XP009532288, ISSN: 2186-5884
- ENOMOTO, NORIYUKI ET AL.: "Clinical study of serum ferritin concentration and anti-MDA5 antibody in acute exacerbation of idiopathic pulmonary fibrosis", ANNALS OF THE JAPANESE RESPIRATORY SOCIETY, vol. 5, no. Suppl., 29 February 2016 (2016-02-29), pages 272, XP009532287, ISSN: 2186-5876
- LI YANAN, BAO JING, BIAN YANGYANG, ERBEN ULRIKE, WANG PEIGANG, SONG KUN, LIU SHUANGQING, LI ZHENZHEN, GAO ZHANCHENG, QIN ZHIHAI: "S100A4+ Macrophages Are Necessary for Pulmonary Fibrosis by Activating Lung Fibroblasts", FRONTIERS IN IMMUNOLOGY, vol. 9, no. 1776, 6 August 2018 (2018-08-06), XP055771000, DOI: 10.3389/fimmu.2018.01776
- AKIYAMA NORIMICHI, HOZUMI HIRONAO, ISAYAMA TAKUYA, OKADA JUN, SUGIURA KATSUNORI, YASUI HIDEKI, SUZUKI YUZO, KONO MASATO, KARAYAMA : "Clinical significance of serum S100 calcium-binding protein A4 in idiopathic pulmonary fibrosis", RESPIROLOGY, vol. 25, no. 7, 9 October 2019 (2019-10-09) - July 2020 (2020-07-01), pages 743 - 749, XP055771012

## Description

### [Technical Field]

The present invention relates to a method for predicting a prognosis of idiopathic pulmonary fibrosis.

### [Background Art]

Idiopathic pulmonary fibrosis (IPF) is a very frequent chronic respiratory disease that accounts for about a half of interstitial pneumonia of unknown cause (idiopathic interstitial pneumonia). This disease is characterized by the progression of fibrosis of the lung and irreversible respiratory dysfunction caused by this, and has such a very poor prognosis that the median survival time from diagnosis is about 2 to 3 years. Although its detailed pathophysiology has not been sufficiently clarified, it has been suggested that the repetitive injury of the alveolar epithelium and excessive tissue repair, mainly by fibroblasts and myofibroblasts are involved in the progression of fibrosis of the lung.

The current guidelines recommend antifibrotic drugs (pirfenidone, nintedanib) as treatments for IPF patients, which have already been covered by insurance in Japan. These therapeutic drugs are useful in suppressing the progression of the disease, that is, deterioration in respiratory functions, but it is difficult with these therapeutic drugs to ameliorate the fibrosed lungs which have already progressed to high degrees. Lung transplantation can also be considered as another treatment, but its adaptation is not widely available, and there is also a problem that the waiting periods for transplantation are very long, about 2 to 3 years in Japan. Hence, it has been considered that for IPF patients, therapeutic interventions in early stages are important to ameliorate the prognoses of the IPF patients.

However, the clinical courses of IPF patients include a wide variety of courses, that is, not only patients whose symptoms progress on a monthly basis and patients whose symptoms deteriorate stepwise but also patients whose symptoms deteriorate on a yearly basis, patients whose symptoms are stable for a long period of time with no treatment, patients whose symptoms suddenly exacerbate after a stable course for a long period of time, and the like. Both of the antifibrotic drugs and the lung transplantation sometimes bring about problems of risk of treatment-related complications, decrease in quality of life, and expensive health care costs, and the timing of the therapeutic intervention requires careful consideration by specialist. Currently in Japan, as a serum biomarker that assists the diagnosis of interstitial pneumonia, Krebs von den Lungen-6 (KL-6) is listed for insurance. However, this KL-6 cannot be said to be necessarily useful for prognosis prediction <NPL 1>, the clinical significance of KL-6 as a criterion for an indication for treatment is unclear.

Hence, the development of a convenient criterion that allows an indication for treatment to be determined appropriately in an early stage in clinical practices, that is, a highly precise biomarker that allows an IPF patient having a risk of poor prognosis to be found in an early stage has been desired. Akiyama et al.: "Clinical Utility of Serum S100 Calcium Binding Protein A4 in Idiopathic Pulmonary Fibrosis", THE AMERICAN REVIEW OF RESPIRATORY DISEASE, AMERICAN THORACIC SOCIETY, US, vol. 199, no. 9, 22 May 2019 (2019-05-22), page A7131, discloses methods and entities suitable for determining the prognosis of idiopathic pulmonary fibrosis based on the expression of biomarkers.

### [Citation List]

### [Non Patent Literature]

[NPL 1] Barratt SL et al, Journal of Clinical Medicine, 2018, August 6, vol. 7, No. 8, pii: E201.

### [Summary of Invention]

### [Technical Problem]

The present invention has been made in view of the above-mentioned problems of the conventional techniques, and an object thereof is to provide a method for determining a risk of poor prognosis of idiopathic pulmonary fibrosis with high precision.

### [Solution to Problem]

In order to achieve the above object, the present inventors studied relevance between the amount of each protein and the progression of the disease (deterioration in respiratory function) and the mortality rate (life prognosis) in clinical samples (serums and lung tissues) of idiopathic pulmonary fibrosis patients. As a result, the present inventors found that 3 proteins S100A4, CIRP, 14-3-3γ were strongly expressed in the lung tissues in the IPF patients as compared with the healthy control (HC). In addition, the concentration of each protein (serum levels) in the serums also indicated significantly high values in the IPF patient as compared with HC. Moreover, the present inventors found that the serum level of each protein was relevant to poor prognoses (progression of the disease and mortality rate) of the IPF patients, and consequently completed the present invention. More specifically, the present invention relates to the following.
<1> A method for determining a prognosis of idiopathic pulmonary fibrosis, comprising the following steps (a) to (c):
   (a) a step of detecting an amount of cold-inducible RNA binding protein (CIRP), for a biological sample separated from the test subject;
   (b) a step of comparing the amount of CIRP detected in the step (a) with a standard amount of CIRP and
   (c) a step of determining that the prognosis of idiopathic pulmonary fibrosis of the test subject is poor in a case where as a result of the comparison in the step (b), the amount of CIRP in the test subject is higher than the standard amount.
<2> The method according to <1>, wherein
   the biological sample is a serum.
<3> Use of an antibody that binds to CIRP for determining a prognosis of idiopathic pulmonary fibrosis by the method according to <1> or <2>,
<4> Use of a kit comprising an antibody that binds to CIRP; and at least one article selected from an isotype control antibody for the antibody, a positive control, and a negative control, for determining a prognosis of idiopathic pulmonary fibrosis.

### [Advantageous Effects of Invention]

The present invention makes it possible to determine the risk of poor prognosis of idiopathic pulmonary fibrosis with high precision. Particularly, even when the serum level of a biomarker is used as a criterion, it is possible to determine the poor prognosis of idiopathic pulmonary fibrosis with high precision. Hence, the present invention also makes it possible to easily determine the prognosis of idiopathic pulmonary fibrosis with low invasiveness and with high precision.

### [Brief Description of Drawings]

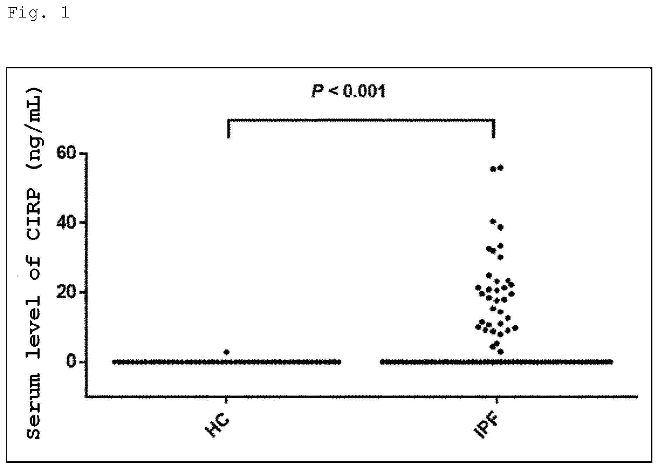

[Fig. 1] Fig. 1 is a graph showing a result of comparing healthy control (HC) and idiopathic pulmonary fibrosis patients (IPF) for serum CIRP level.

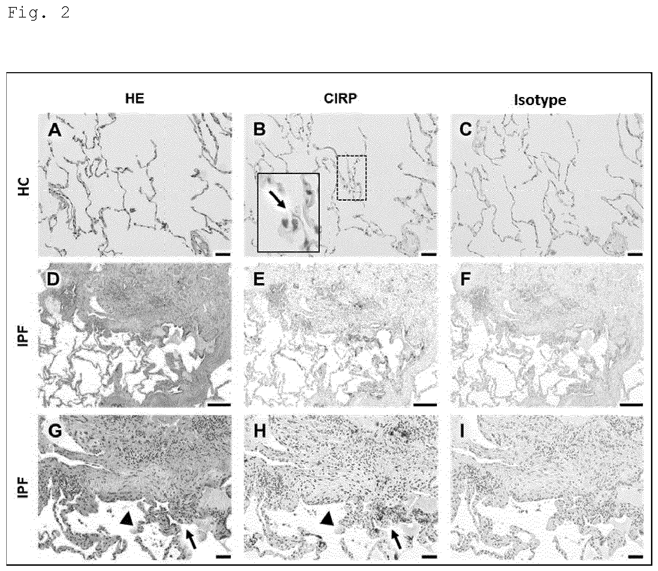

[Fig. 2] Fig. 2 is pictures showing a result of observing the lung tissues of the healthy control (HC) and the idiopathic pulmonary fibrosis patients (IPF) with microscopy. In Fig. 2, "HE" indicates a result of analysis with HE staining, "CIRP" indicates a result of analysis with immunostaining using an anti-CIRP antibody, and "Isotype" indicates a result of analysis with immunostaining using an isotype control antibody. The scale bar represents 50 µm in A, B, C, G, H, and I, and represents 500 µm in D, E, and F.

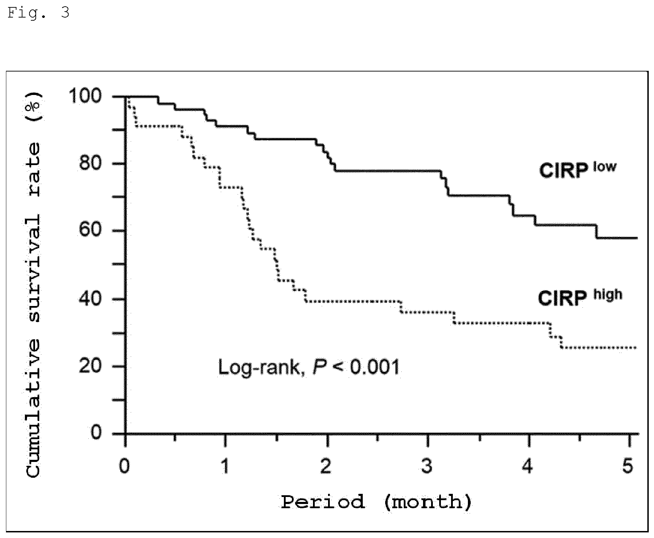

[Fig. 3] Fig. 3 is a graph showing a result of comparing a high serum CIRP-value group (CIRP^{high}) and a low-value group (CIRP^{low}) in idiopathic pulmonary fibrosis patients for survival rates. In Fig. 3, the vertical axis indicates a cumulative survival rate and the horizontal axis indicates the number of years elapsed after the sampling of serums.

### [Description of Embodiments]

### <Method for Determining Prognosis of Idiopathic Pulmonary Fibrosis>

As indicated in Examples described below, as a result of studying relevance between the amount of protein and the progression of the disease (deterioration in respiratory function) and the mortality rate (life prognosis) in the clinical samples (serums and lung tissues) of idiopathic pulmonary fibrosis patients, the present inventors found that the protein, CIRP, was strongly expressed in the lung tissues in the IPF patients as compared with healthy control (HC). Moreover, the present inventors also found possibilities that fibroblasts in the lung tissues of the IPF patients acted as a production source of the protein, and the expression level reflected the fibrogenesis activities of the lungs and was involved in the progression of the disease.

In addition, the present inventors revealed that the serum level of the protein in the serums indicated a significantly high value in the IPF patients as compared with HC, and further found that the serum level of each protein is relevant to poor prognoses (progression of the disease and mortality rate) of the IPF patients.

The present invention has been completed based on the above findings, and provides a method for determining a prognosis of idiopathic pulmonary fibrosis, comprising the following steps (a) to (c):
(a) a step of detecting an amount of CIRP in a biological sample separated from a test subject affected with idiopathic pulmonary fibrosis;
(b) a step of comparing the amount of the protein detected in the step (a) with a standard amount of the corresponding protein; and
(c) a step of determining that the prognosis of the idiopathic pulmonary fibrosis of the test subject is poor in a case where the amount of the protein in the test subject is higher the standard amount as a result of the comparison in the step (b).

"Idiopathic pulmonary fibrosis (IPF)" is a chronic respiratory disease, which is a kind of interstitial pneumonia of unknown cause (idiopathic interstitial pneumonia), and is characterized by the progression of fibrosis of the lung and irreversible respiratory dysfunction caused by this. In the present invention, the "prognosis of idiopathic pulmonary fibrosis" means the future state of idiopathic pulmonary fibrosis in the test subject after the biological sample is separated. In addition, the "poor prognosis of idiopathic pulmonary fibrosis" means that the symptoms of idiopathic pulmonary fibrosis progress fast and includes a low survival rate brought by the symptom progression. Here, the "symptoms" include, for example, respiratory dysfunction, oxygenation defects, and a decrease in exercise tolerance. More specifically, the "symptoms" include a decrease in forced vital capacity (FVC) by 10% or more, a decrease in diffusing capacity of the lung for carbon monoxide (DLCO) by 15% or more, and a decrease in 6-minute walk distance by 50 m or more. The "Fast progress" includes, for example, a case where the symptoms occur within 1 year after the separation of the biological sample. In addition, the "low survival rate" includes, for example, a case where the survival rate (cumulative survival rate) within 2 years becomes 60% or less after the separation of the biological sample.

In the present invention, the "test subject" is not particularly limited as long as the "test subject" is a human, but is normally a human affected with idiopathic pulmonary fibrosis. Note that the test subject includes not only a test subject that has not received treatment of idiopathic pulmonary fibrosis at the time of separation of the biological sample but also a test subject that has received treatment at that time.

The "biological sample separated from the test subject" may be a sample (a cell, a tissue, an organ, body fluids (blood, lymph, and the like), digestive juice, induced sputum, bronchoalveolar lavage fluid, urine, feces, and the like) extirpated from the test subject (living organism of a human) and completely separated from the living organism from which the sample is derived, and may preferably be blood (serum, plasma, or the like), lung tissue, induced sputum, or bronchoalveolar lavage fluid. Moreover, the blood is more preferable, and serum is further preferable, from the viewpoint that they can be separated from the test subject with low invasiveness and allows preparation of a protein and detection of the expression level of the protein to be easily conducted. In addition, in the case of subjecting the biological sample to a method for detecting the amount of a protein described later, the biological sample may further be prepared into a form suitable for the method (for example, a protein solution extracted from the biological sample, a tissue subjected to the formalin fixation process, the alcohol fixation process, the freezing process, or the paraffin embedding process, or the like) as appropriate. A person skilled in the art can select a publicly-known approach for preparation taking the type, state, and the like of the biological sample into consideration.

"CIRP" to be detected in the present invention is a protein also called cold-inducible RNA binding protein (CIRBP), and is typically a protein having an amino acid sequence identified by Ref Seq ID: NP_001271, NP_001287744, or NP_001287758 when it is derived from human.

The DNA sequence of a gene encoding a protein can mutate in the natural world (that is, non-artificially) in accordance with the mutation, or the like. Hence, the aforementioned proteins to be detected in the present invention are not specified to the above-described typical amino acid sequences, but also include natural mutants of these amino acid sequences. In addition, the aforementioned proteins to be detected in the present invention include not only those having amino acid sequences of full lengths but also partial peptides thereof.

The "amount of the protein" to be detected in the present invention may be not only an absolute amount but also a relative amount. The relative amount includes, for example, a protein amount ratio (a numerical value represented by a so-called arbitrary unit (AU)) based on a measuring method or a measuring device used for detection. Alternatively, as the relative amount, for example, a value calculated based on an amount of a reference protein may be used. The "reference protein" according to the present invention only has to be a protein that is stably present in a biological sample and has a small difference in amount between different biological samples, and includes, for example, endogenous control (internal standard) proteins, and more specifically includes β-actin, α-tubulin, COX-4, GAPDH, lamin B1, PCNA, TBP, VDCA1/Porin.

The "detection of the amount of the protein" can be performed by a person skilled in the art employing a publicly-known approach as appropriate. The publicly-known approach includes, for example, methods for detection using antibodies (immunological approaches) such as the enzyme-linked immunosorbent assay (ELISA method), the CLEIA(chemiluminescent enzyme immunoassay method), the latex agglutination method, the antibody array, the immunoblotting, the immunochromatography, the imaging cytometry, the flow cytometry, the radioimmunoassay, the immunoprecipitation method, and the immunohistochemical staining method, and the mass analysis method.

In the immunological approach, an antibody that binds to CIRP is used, and the antibody is brought into contact with proteins (target proteins) to which the antibody binds, to detect the amount of each protein based on the binding properties of the antibody to the respective protein as a criteria.

More specifically, in the ELISA method (sandwich ELISA method), first, CIRP in the biological sample is brought into contact with an antibody that binds to CIRP which is fixed to a substrate (capture antibody) to capture the target protein corresponding to the antibody. Subsequently, an anti-CIRP antibody or the like (detecting antibody) with a marker described later is reacted with the captured CIRP or the like, and the marker is chemically or optically detected, making it possible to detect the amount of each target protein.

Note that the "capture antibody" and the "detecting antibody" may be the same antibody or may be different antibodies as long as these antibodies identify each target protein. However, the "capture antibody" and the "detecting antibody" are preferably different antibodies from the viewpoint that this makes it possible to noncompetitively capture and detect each target protein. Such different antibodies may be, for example, a combination in which the capture antibody is a polyclonal antibody for the target protein and the detecting antibody is a monoclonal antibody for the same target protein, a combination in which the capture antibody is a monoclonal antibody for the target protein and the detecting antibody is a polyclonal antibody for the same target protein, or a combination in which the capture antibody is a monoclonal antibody for the target protein and the detecting antibody is a monoclonal antibody for a target protein recognizing a site (epitope) different from the site recognized by the capture antibody.

In addition, besides the method for directly measuring the amount of an antibody that binds to the target protein by using an antibody to which a labeling substance is bound, an indirect detecting method such as a method utilizing a secondary antibody to which a labeling substance is bound or a method utilizing a polymer in which a secondary antibody and a labeling substance are bound. Here, the "secondary antibody" is an antibody that exhibits a specific binding ability to the antibody according to the present invention. Alternatively, it is possible to use protein G, protein A, or the like to which a labeling substance is bound instead of the secondary antibody.

In the determining method of the present invention, the amount of the protein detected in this way and a standard amount of the same protein are compared. It is possible for a person skilled in the art to conduct such comparison by selecting a statistical analysis method suitable for the above-described detecting method as appropriate. The statistical analysis method includes, for example, the t-test, the analysis of variance (ANOVA), the Kruskal-Wallis test, the Wilcoxon test, the Mann-Whitney test, the odds ratio, the hazard ratio, the Fisher's exact test, the Receiver Operating Characteristic analysis (ROC analysis), and the classification and decision trees analysis (CART analysis). In addition, in the comparison, normalized or standardized and normalized data may also be used.

The "standard amount of the protein" to be compared is not particularly limited, but can be set by a person skilled in the art as a so-called cutoff value based on which it can be determined whether the prognosis of idiopathic pulmonary fibrosis is poor or not in conformity with the above-described detecting method and statistical analysis method.

The standard amount may be a value set to divide the amounts of each protein detected in a plurality of idiopathic pulmonary fibroses into a high-value group and a low-value group as shown in Examples described later. The standard amount may be a median or an average value of amounts of each protein detected in a plurality of idiopathic pulmonary fibroses. The standard amount may be a value determined by comparing amounts of each protein between a patient group with poor prognosis of idiopathic pulmonary fibrosis and a patient group without it. The standard amount of CIRP may be, for example, 0.2 ng/mL.

Whether an amount is "higher than the standard amount of the protein" can be judged by a person skilled in the art based on the above-described statistical analysis method as appropriate. For example, such a case includes one in which the amount of the protein detected is higher than the corresponding standard amount and the difference is statistically significant (for example, P<0.05). In addition, for example, such a case also includes one in which the amount of the protein detected is twice or more the corresponding standard amount.

In addition, the determination on the prognosis of idiopathic pulmonary fibrosis is normally conducted by a doctor (including someone instructed by a doctor), but data on the aforementioned amount of the protein and the like is useful for a diagnosis including judgment on the timing of treatment by a doctor and the like. Thus, the method of the present invention can be expressed as a method for collecting data on the aforementioned amount of the protein for the determination on prognosis (diagnosis) by a doctor, a method for presenting the data to a doctor, a method for comparing and analyzing the aforementioned amount of the protein with the corresponding standard amount of the protein, or a method for assisting the determination on prognosis by a doctor.

### <Drug for Determining Prognosis of Idiopathic Pulmonary Fibrosis>

As mentioned above, in the determining method of the present invention, it is possible to determine the prognosis of idiopathic pulmonary fibrosis by detecting an amount of CIRP protein using an antibody that binds to the target protein.

Hence, the present invention provides a drug for determining a prognosis of idiopathic pulmonary fibrosis by the aforementioned method, the drug comprising at least an antibody that binds to CIRP.

The "antibody" contained in the drug of the present invention may be a polyclonal antibody or may be a monoclonal antibody, or may be a functional fragment of an antibody. The "antibody" includes all the classes and subclasses of immunoglobulins. The "polyclonal antibody" is an antibody preparation containing different antibodies for different epitopes. In addition, the "monoclonal antibody" means an antibody (including an antibody fragment) obtained from a group of substantially uniform antibodies. In contrast to the polyclonal antibody, the monoclonal antibody recognizes a single determinant on an antigen. In the present invention, the "functional fragment" of an antibody means a part (partial fragment) of the antibody, that specifically recognizes the target protein. Specifically, the "functional fragment" includes Fab, Fab', F(ab')2, variable region fragment (Fv), disulfide bonded Fv, single chain Fv (scFv), sc(Fv)2, diabody, polyspecific antibodies, and polymers of these, and the like.

When the antibody according to the present invention is a polyclonal antibody, the polyclonal antibody can be obtained by immunizing an immune animal using an antigen (CIRP, partial peptides of this, cells expressing this, or the like), and purifying the antiserum thereof by a conventional means (for example, salting-out, centrifugation, dialysis, column chromatography, or the like).

In addition, the monoclonal antibody can be prepared by a hybridoma method or a recombinant DNA method. The hybridoma method includes the method of Kohler and Milstein (Kohler & Milstein, Nature, 256: 495 (1975)) as a representative. The recombinant DNA method is an approach that includes cloning a DNA encoding the above-described antibody according to the present invention from a hybridoma, a B cell, or the like, incorporating the DNA into an appropriate vector, introducing the vector into a host cell (for example, a mammalian cell line, E. coli, a yeast cell, an insect cell, a plant cell, or the like) to produce the antibody according to the present invention as a recombinant antibody (for example, P. J. Delves, Antibody Production: Essential Techniques, 1997 WILEY, P. Shepherd and C. Dean Monoclonal Antibodies, 2000 OXFORD UNIVERSITY PR ES S, Vandamme A. M. et al., Eur. J. Biochem. 192: 767-775 (1990)).

The antibody may be provided in a form fixed onto a substrate such as a plate for use in the ELISA method, the antibody array, and the like. In conformity with the above-described detecting method, the antibody may be labeled with a marker substance. The marker substance includes, for example, enzymes such as β-D-glucosidase, luciferase, and HRP, luminescent substances such as luminol, luciferin, and lucigenin, fluorescent substances such as FITC, FAM, DEAC, R6G, TexRed, and Cy5, radioisotopes such as ³H, ¹⁴C, ³²P, ³⁵S, and ¹²³I, and affinity substances such as biotin and streptavidin.

The drug of the present invention may contain an additional component acceptable as a composition besides the above antibody. Such an additional component includes, for example, a pharmacologically acceptable carrier or diluent (sterile water, saline, a vegetable oil, an excipient, a disintegrant, a buffering agent, an emulsifier, a suspension, a stabilizer, a preservative, an antiseptic, and the like). As the excipient, lactose, starch, sorbitol, D-mannitol, white soft sugar, or the like can be used. As the disintegrant, starch, carboxymethyl cellulose, calcium carbonate, and the like can be used. As the buffering agent, phosphoric acid salts, citric acid salts, acetic acid salts, and the like can be used. As the emulsifier, gum arabic, sodium alginate, tragacanth, and the like can be used. As the suspension, glyceryl monostearate, aluminum monostearate, methyl cellulose, carboxymethyl cellulose, hydroxymethyl cellulose, sodium lauryl sulfate, and the like can be used. As the stabilizer, propylene glycol, diethylin sulfite, ascorbic acid, and the like can be used. As the preservative, phenol, benzalkonium chloride, benzyl alcohol, chlorobutanol, methylparaben, and the like can be used. As the antiseptic, sodium azide, benzalkonium chloride, parahydroxybenzoic acid, chlorobutanol, and the like can be used.

In addition, besides the above-described antibody (antibody that binds to CIRP or the like) or drug of the present invention, a substrate necessary for detecting a marker, a solution for dissolving a protein of a biological sample (reagent for dissolving proteins), a buffer solution used for diluting or washing a sample (dilute solution, cleaning liquid), a reagent for stopping detection reaction of a marker (reaction stopping agent), a positive control (for example, each target protein, preparation, or a biological sample derived from an idiopathic pulmonary fibrosis patient with a poor prognosis), a negative control (for example, a biological sample derived from an idiopathic pulmonary fibrosis patient without a poor prognosis), an isotype control antibody for the antibody (antibody that binds to S100A4 or the like) according to the present invention, and the like may be combined to obtain a kit for determining a prognosis of idiopathic pulmonary fibrosis. Such a kit includes, for example, a kit for determining a prognosis of idiopathic pulmonary fibrosis, comprising: at least one antibody that binds to CIRP, and at least one article selected from an isotype control antibody for the antibody, a positive control, and a negative control. In addition, in a case where an unlabeled antibody is used as an antibody preparation, a substance (for example, a secondary antibody, protein G, protein A, or the like) that binds to the antibody, which has been labeled may be combined. Moreover, such a kit may contain an instruction manual for the kit.

### <Method for Treating Idiopathic Pulmonary Fibrosis>

The clinical courses of idiopathic pulmonary fibrosis patients include a wide variety of courses, that is, not only patients whose symptoms progress on a monthly basis and patients whose symptoms deteriorate stepwise but also patients whose symptoms deteriorate on a yearly basis, patients whose symptoms are stable for a long period of time with no treatment, patients whose symptoms suddenly exacerbate after a stable course for a long period of time, and the like. Both of antifibrotic drugs and lung transplantation sometimes bring about problems of risk of treatment-related complications, decrease in quality of life, and expensive health care costs. For this reason, the method of the present invention that makes it possible to determine the prognosis of idiopathic pulmonary fibrosis of the present invention to conduct a timely treatment is very effective in conducting the treatment of the idiopathic pulmonary fibrosis. It is possible to start treatments on patients with poor prognosis which has not been identified using an existing marker or through clinical observation using an antifibrotic drug in an early stage or when the symptoms are still mild, and effects such as extension of lifetime or avoidance of becoming severe can be expected.

Hence, also described herein is a method for treating idiopathic pulmonary fibrosis, comprising administering a therapeutic drug for idiopathic pulmonary fibrosis to a test subject whose prognosis of idiopathic pulmonary fibrosis has been determined to be poor by the method of the present invention, and/or, performing lung transplantation on the test subject.

The time to start administering a therapeutic drug or propose lung transplantation or start preparing for lung transplantation is preferably within 6 months, more preferably within 3 months, further preferably within 2 months, and particularly preferably within 1 month (for example, within 3 weeks, within 2 weeks, or within 1 week) after separation of the above-described biological sample, from the viewpoint of suppressing the progression of idiopathic pulmonary fibrosis in an early stage and improving the prognosis.

Although the method for administering a therapeutic drug is different depending on the type and formulation of the therapeutic drug, the age, body weight, gender, and the like of a test subject to be administered, the therapeutic drug may be administered any administration route out of oral administration and parenteral administration (for example, intravenous administration, intraarterial administration, or topical administration). The dosage may be adjusted by a person skilled in the art in accordance with the type and formulation of the therapeutic drug, the age, body weight, gender, health status, and the like of the test subject to be administered as appropriate (in the case of oral administration, the dosage is 0.1 to 100 mg, preferably 1 to 50 mg a day per 1 kg body weight for adults.)

The "therapeutic drug for idiopathic pulmonary fibrosis" only has to be a drug having actions related to suppression of the progression of symptoms of idiopathic pulmonary fibrosis and alleviation of symptoms, and includes, for example, antifibrotic drugs, immunosuppressants, and steroid drugs. More specifically, the antifibrotic drugs include TGF-βproduction inhibitors such as pirfenidone and tyrosine kinase inhibitors such as nintedanib. The immunosuppressants include alkylating agents such as cyclophosphamide, antimetabolites such as azathioprine, and calcineurin inhibitors such as cyclosporin. The steroid drugs includes corticosteroid-based drug (glucocorticoid-based drug) such as prednisolone and methylprednisolone.

In addition, according to the present invention, the usage (administration target, administration period) of the therapeutic drug is specified. Hence, the present invention also provides a therapeutic drug for idiopathic pulmonary fibrosis to be administered to a test subject whose prognosis of idiopathic pulmonary fibrosis has been determined to be poor by the method of the present invention.

### [Examples]

Although the present invention is described below in more detail based on Examples, the present invention is not limited to the following Examples. In addition, Examples were conducted using methods and ingredients shown below.

### <Study on Serum Biomarkers in Idiopathic Pulmonary Fibrosis (IPF) Patients>

### (Target·Diagnosis)

Targets were 95 untreated IPF patients and 50 healthy subjects (HC) statistically having no difference in age·gender distribution. IPF was diagnosed based on the international guidelines (see American journal of respiratory and critical care medicine. 2002; 165: 277-304., Travis WD et al., American journal of respiratory and critical care medicine. 2013; 188: 733-48., Raghu G et al., American journal of respiratory and critical care medicine. 2011; 183: 788-824., Raghu G et all., American journal of respiratory and critical care medicine. 2018; 198: e44-e68.). The acute exacerbation of IPF (AE-IPF) was diagnosed based on the criteria of the 2016 International Working Group (see Collard HR et al., American journal of respiratory and critical care medicine. 2016; 194: 265-75.).

### (Design of Study)

In this study, the serum was sampled from each above-described target, and the serum levels of the biomarker (CIRP) were measured. The date of sampling the serums was set as the beginning date, and relevance between these biomarker values and clinical parameters measured from the ages, genders, and the beginning date within 1 week from the beginning date as well as the progression of the disease (deterioration in the respiratory functions) from the beginning date and death was retrospectively analyzed. The progression of the disease within 1 year from the beginning date (deterioration in respiratory functions in which 10% or more of %FVC decreased within 1 year from the beginning date) or death was defined as "poor prognosis". The lifetime was calculated as days from the beginning date to a death event or the last date of confirmation of survival.

### (Measurement of Clinical Parameters)

The serum Krebs von den Lungen-6 (KL-6) value was measured by the ECLIA method (Nanopia (trade mark) KL-6, manufactured by SEKISUI MEDICAL CO., LTD.) using the venous blood serum (serum) sampled from each patient.

The arterial oxygen pressure (PaO₂) was measured by a blood gas analyzer (Rapidpoint 500, manufactured by Siemens Healthcare Diagnostics Manufacturing Ltd) using the arterial blood sampled from the radial artery, brachial artery, or femoral artery of each patient who maintained the rest for 15 minutes under room air inhalation.

The forced vital capacity (FVC) was measured by a spirometer (DISCOM-21 FXIII, manufactured by CHEST M.I., INC.), and %FVC was calculated as a percentage of FVC to the predicted vital capacity.

The diffusing capacity of the lung for carbon monoxide (DLCO) was measured by a precise respiratory function testing device ( CHESTAC-8900, manufactured by CHEST M.I., INC.), and DLCO was calculated as a ratio of the actual DLCO to the predicted DLCO.

### (Immunostaining)

Immunostaining was performed using formalin-fixed specimens sampled from the IPF patients through surgical lung biopsy. A formalin-fixed specimen of a part of the healthy lung excised from a lung cancer patient who was not of IPF was used as a comparison subject. Sections having a thickness of 5 µm were made from these specimens and subjected to a deparaffinization process, and then were heated for 30 minutes using a citric acid buffer of pH 6.0. Then, these sections were reacted with a 3% hydrogen peroxide solution for 15 minutes to perform the process of blocking endogenous peroxidase. Next, the sections were reacted with primary antibodies (an anti-S100A4 antibody, an anti-CIRP antibody, and an anti-14-3-3γ antibody), which are described later, for 1 hour at room temperature, and thereafter, were reacted with an immunohistochemical staining reagent (Histofine Simple Stain MAX-PO(M), manufactured by Nichirei Corp) for 30 minutes. The immunoreaction on each section was visualized with a 3,3-diaminobenzidine dye and was further subjected to nuclear stain with hematoxylin:
Anti-CIRP antibody: rabbit-derived anti-human CIRP antibody (ab191885), manufactured by Abcam, 1000-fold dilution

### (Statistics)

The continuous variable was expressed with the median and interquartile range (IQR). The qualitative variable was expressed with the number (n) and percent (%). For the comparison between the groups, the Wilcoxon/Kruskal-Wallis test and the Fisher's exact test were used. The correlations between the clinical parameters and, CIRP were analyzed using the Spearman's correlation test. The cumulative lifetime was calculated by the Kaplan-Meier method. For the comparison of survival rate between the groups, the log-rank test was used. For the risk factor analysis on the progression of the disease, the logistic regression analysis was used. At this time, the multivariate analysis was performed using all the clinical parameters that indicated significant relevance with the progression of the disease in the univariate logistic analysis. The risk factor of the death event from the beginning date (poor life prognosis factor) was calculated by the Cox proportional hazard model using lifetime. At this time, the multivariate analysis was performed using all the clinical parameters that indicated significant relevance with the death event in the univariate Cox proportional hazard analysis. In this study, P-value<0.05 was determined to be statistically significant. For the statistical analysis, software such as JMP version 13.2.1 (SAS Institute Inc) and EZR version 1.38 (Jichi Medical University) was used.

The results of analyses using the above methods, materials, and the like are shown below for the biomarker.

### (Example 1) <Study on CIRP in IPF Patients>

### [Comparison of Serum CIRP between Healthy Control and IPF Patients]

The CIRP values of serums sampled from 95 untreated IPF patients and 50 subjects of healthy control (HC) having no statistical difference in age·gender distribution from the IPF patients were measured using the ELISA method (CY-8103 Human CIRP ELISA Kit (manufactured by MEDICAL & BIOLOGICAL LABORATORIES CO., LTD.)). As a result, as shown in Fig. 4, the serum CIRP values of the IPF patient group were significantly higher than those of the HC group. Interestingly, the serum CIRP values of most of HC were equal to or lower than the measurement sensitivity of the present kit (0.201 ng/mL). While there was a patient group having serum CIRP values equal to or lower than the measurement sensitivity in the IPF patient group as well, there was also a group having serum CIRP values equal to or higher than the measurement sensitivity. Hence, the former was defined as a low CIRP-value group and the latter was defined as a high CIRP-value group, and these were compared later.

### [Comparison of CIRP Expression in lung tissue between Healthy Control and IPF Patients]

With the normal lung tissue sites in lung cancer patients as a healthy control (HC), the expression of CIRP in the lung tissues of IPF patients obtained by surgical lung biopsy were compared and studied using the immunostaining method. As a result, as shown in Fig. 1, in HC, weak expression of CIRP was observed in part of the normal alveolar structure (arrow in B of Fig. 1). In contrast, in the lung tissues of the IPF patients, diffuse and strong expression of CIRP was observed (E of Fig. 1). Particularly, CIRP was strongly expressed in fibroblastic foci (arrowhead in H of Fig. 1) and the periphery of the fibrotic tissue and in the nuclei of proliferated cells (arrow in H of Fig. 1). The above observation suggested a possibility that the fibrotic region in the lung tissues of the IPF patients were the production source of CIRP, the expression level of CIRP reflected the fibrogenesis activity of the lung, and thus CIRP is involved in the progression of the disease.

### [Correlation between Serum CIRP and Clinical Parameters]

The correlation between the serum CIRP values and the clinical parameters in the IPF patients was studied. As shown in Table 1, no significant correlation was observed. The serum CIRP values showed a behavior independent from the existing clinical parameters.

**[Table 1]**

| Characteristics | Correlation coefficient | *P*-value |
|---|---|---|
| Age | -0.02 | 0.97 |

| Laboratory parameters | | |
|---|---|---|
| KL-6, U/mL | -0.03 | 0.93 |
| PaO₂, Torr | 0.07 | 0.15 |

| Laboratory parameters on lung functions | | |
|---|---|---|
| %FVC | -0.25 | 0.5 |
| %DLCO | -0.13 | 0.73 |

### [Comparison between High Serum CIRP-value Group and Low-value Group in IPF Patients]

Baseline characteristics were compared between the high CIRP-value group and the low-value group. As shown in Table 2, %FVC was low in the high CIRP-value group, but there was no other significant difference. However, the poor prognosis rate was significantly higher in the high CIRP-value group than in the low-value group.

**[Table 2]**

| | CIRP^{high}, n=35 | CIRP^{low}, n=60 | *P*-value |
|---|---|---|---|
| Age | 72 (64-78) | 70 (65-75) | 0.47 |
| Male/Female | 29 (83)/6 (17) | 54 (90)/6 (10) | 0.35 |
| KL-6, U/mL | 924 (519-1471) | 941 (599-1317) | 0.99 |
| PaO₂, Torr | 78 (67-88) | 76 (69-84) | 0.8 |
| %FVC, % | 63 (52-82) | 80 (64-92) | <0.01* |
| %DLCO | 63 (37-84) | 62 (46-86) | 0.62 |
| Progression of disease and death within 1 year | 19 (54.3) | 16 (26.7) | <0.01* |
| Progression of disease (deterioration in lung functions) | 10 (28.6) | 10 (16.7) | 0.1 |
| Death | 9 (26.5) | 6 (10.3) | 0.08 |

In addition, the survival rate was compared between the high serum CIRP-value group and the low-value group in the IPF patients. As shown in Fig. 2, the life prognosis of the high CIRP-value group was significantly poor as compared with the low-value group. Note that the two-year survival rate after the IPF diagnosis was 39.5% in the high CIRP-value group and 83.9% in the low CIRP-value group.

### [Relevance of serum CIRP with prognosis in IPF Patients]

Relevance between the serum CIRP value and the progression of the disease within 1 year from the beginning date in the IPF patients was analyzed by the logistic regression analysis. As shown in Table 3, low PaO₂ value, low %FVC value, and high serum CIRP value were significantly relevant to the progression of the disease in univariate analysis. Moreover, when multivariate analysis was performed using these variables that were significant in the univariate analysis, it was found that the high serum CIRP value was an independent risk factor for the progression of the disease.

**[Table 3]**

| | OR | 95% CI | *P*-value |
|---|---|---|---|
| Univariate analysis | | | |
| Female (vs. Male) | 1. 63 | 0.44-6.12 | 0.47 |
| Age | 1.01 | 0.96-1.06 | 0.8 |
| PaO₂, per increase of 1 Torr | 0.96 | 0.93-0.99 | 0.04* |
| %FVC, per increase of 1% | 0.93 | 0.90-0.96 | <0.01* |
| KL-6, per increase of 100 U/mL | 0.99 | 0.95-1.04 | 0.73 |
| Serum CIRP^{high} (vs. CIRP^{low}) | 3.39 | 1.38-8.36 | <0.01* |
| Serum CIRP, per increase of 1 ng/mL | 1.06 | 1.02-1.11 | <0.01* |

| Multivariate analysis model 1 | | | |
|---|---|---|---|
| PaO₂, per increase of 1 Torr | 0.97 | 0.92-1.01 | 0.15 |
| %FVC, per increase of 1% | 0.94 | 0.91-0.97 | <0.01* |
| Serum CIRP, per increase of 1 ng/mL | 1.06 | 1.01-1.11 | 0.01* |

| Multivariate analysis model 2 | | | |
|---|---|---|---|
| PaO₂, per increase of 1 Torr | 0.98 | 0.93-1.02 | 0.26 |
| %FVC, per increase of 1% | 0.94 | 0.91-0.97 | <0.01* |
| Serum CIRP^{high} (vs. CIRP^{low}) | 2.27 | 0.79-6.49 | 0.13 |

Note that in Table 3, "OR" indicates an odds ratio and "95% CI" indicates a 95% confidence interval. In addition, numerical values attached with asterisk indicate P<0.05.

Next, relevance between the serum CIRP value and the clinical parameters and poor life prognosis of deceased patients was analyzed using the Cox proportional hazard model. As shown in Table 4, advanced age, low PaO₂ value, low %FVC value, and high serum CIRP value were significantly relevant to the poor life prognosis in univariate analysis. When multivariate analysis was performed using these variables that were significant in the univariate analysis, the high serum CIRP value was an independent poor life prognosis factor. On the other hand, KL-6 which has already been used in daily medical practice was not relevant to poor prognosis.

**[Table 4]**

| | HR | 95% CI | *P*-value |
|---|---|---|---|
| Univariate analysis | | | |
| Female (vs. Male) | 0.65 | 0.30-1.71 | 0.35 |
| Age | 1.04 | 1.01-1.09 | 0.02* |
| PaO₂, per increase of 1 Torr | 0.94 | 0.91-0.97 | <0.01* |
| %FVC, per increase of 1% | 0.95 | 0.93-0.96 | <0.01* |
| KL-6, per increase of 100 U/mL | 1.02 | 0.99-1.04 | 0.19 |
| Serum CIRP, per increase of 1 ng/mL | 1.03 | 1.01-1.04 | 0.01* |
| Serum CIRP^{high} (vs. CIRP^{low}) | 2.86 | 1.64-5.05 | <0.01* |

| Multivariate analysis model 1 | | | |
|---|---|---|---|
| Age | 1.02 | 0.98-1.06 | 0.27 |
| PaO₂, per increase of 1 Torr | 0.94 | 0.91-0.97 | <0.01* |
| %FVC, per increase of 1% | 0.96 | 0.94-0.98 | <0.01* |
| serum CIRP, per increase of 1 ng/mL | 1.02 | 1.002-1.05 | 0.03* |

| Multivariate analysis model 2 | | | |
|---|---|---|---|
| Age | 1.02 | 0.98-1.06 | 0.31 |
| PaO₂, per increase of 1 Torr | 0.95 | 0.92-0.97 | <0.01* |
| %FVC, per increase of 1% | 0.96 | 0.94-0.98 | <0.01* |
| Serum CIRP^{high} (vs. CIRP^{low}) | 2.15 | 1.16-4.03 | 0.01* |

Note that in Table 4, "HR" indicates a hazard ratio and "95% CI" indicates a 95% confidence interval. In addition, numerical values attached with asterisk indicate P<0.05.

### [industrial applicability]

As described above, the present invention makes it possible to determine the risk of poor prognosis of idiopathic pulmonary fibrosis with high precision. Particularly, even when the serum level of a biomarker is used as a criterion, it is possible to determine the poor prognosis of idiopathic pulmonary fibrosis with high precision.

Since PaO₂ serves as a criterion for oxygenation in the lung, PaO₂ is important for the determination of severity. However, its measurement requires arterial blood collection, which is more invasive than venous blood collection. In addition, PaO₂ varies depending on the conditions for oxygen administration, it is necessary to measure PaO₂ under strictly set conditions. Although %FVC serves as a prognosis prediction factor, test itself is difficult in severe cases, cases with respiratory distress, and cases with complications such as pneumothorax. Moreover, reproducibility decreases in patients of advanced age and patients having disorders such as deafness.

On the other hand, according to the present invention, since prognosis can be determined even using a venous serum, it is possible to repeatedly test with low invasiveness without exerting a great burden on patient. In this way, the present invention which also makes it possible to easily determine the prognosis of idiopathic pulmonary fibrosis with low invasiveness and with high precision is very useful in the medical field relating to idiopathic pulmonary fibrosis.

## Claims

1. A method for determining a prognosis of idiopathic pulmonary fibrosis, comprising the following steps (a) to (c):
(a) a step of detecting an amount of cold-inducible RNA binding protein (CIRP) for a biological sample separated from the test subject;
(b) a step of comparing the amount of CIRP detected in the step (a) with a standard amount of CIRP; and
(c) a step of determining that the prognosis of idiopathic pulmonary fibrosis of the test subject is poor in a case where as a result of the comparison in the step (b), the amount of CIRP in the test subject is higher than the standard amount.

2. The method according to claim 1, wherein
the biological sample is a serum.

3. Use of an antibody that binds to CIRP for determining a prognosis of idiopathic pulmonary fibrosis by the method according to claim 1 or 2.

4. Use of a kit comprising:
an antibody that binds to CIRP; and
at least one article selected from an isotype control antibody for the antibody, a positive control, and a negative control, for determining a prognosis of idiopathic pulmonary fibrosis.

## Patentansprüche

1. Verfahren zur Bestimmung einer Prognose für eine idiopathische Lungenfibrose, das folgende Schritte (a) bis (c) umfasst:
(a) einen Schritt zum Erfassen einer Menge an kälteinduzierbarem RNAbindendem Protein (Cold-Inducible-RNA-binding Protein, CIRP) in einer von der Testperson entnommenen biologischen Probe;
(b) einen Schritt zum Vergleich der in Schritt (a) erfassten CIRP-Menge mit einer Standardmenge an CIRP; und
(c) einen Schritt, bei dem bestimmt wird, dass die Prognose für eine idiopathische Lungenfibrose der Testperson ungünstig ist, falls infolge des Vergleichs in Schritt (b) die CIRP-Menge bei der Testperson höher ist als die Standardmenge.

2. Verfahren nach Anspruch 1, wobei die biologische Probe ein Serum ist.

3. Verwendung eines Antikörpers, der an CIRP bindet, um eine Prognose einer idiopathischen Lungenfibrose zu bestimmen nach Anspruch 1 oder 2.

4. Verwendung eines Kits, das umfasst:
einen Antikörper, der an CIRP bindet; und
mindestens einen Bestandteil, ausgewählt aus einem Isotyp-Kontrollantikörper für den Antikörper, einer Positivkontrolle und einer Negativkontrolle, um eine Prognose einer idiopathischen Lungenfibrose zu bestimmen.

## Revendications

1. Procédé de détermination d'un pronostic de fibrose pulmonaire idiopathique, comprenant les étapes (a) à (c) suivantes :
(a) une étape de détection d'une quantité de protéine de liaison à l'ARN inductible par le froid (CIRBP, Cold-Inducible RNA Binding Protein) relativement à un échantillon biologique issu du sujet testé,
(b) une étape de comparaison de la quantité de CIRBP détectée lors de l'étape (a) avec une quantité standard de CIRBP, et
(c) une étape de détermination que le pronostic de fibrose pulmonaire idiopathique du sujet testé est défavorable si le résultat de la comparaison de l'étape (b) montre que la quantité de CIRBP présente chez le sujet testé est plus importante que la quantité standard.

2. Procédé selon la revendication 1, dans lequel
l'échantillon biologique est du sérum.

3. Utilisation d'un anticorps qui se lie à la CIRBP pour déterminer un pronostic de fibrose pulmonaire idiopathique par le biais du procédé selon la revendication 1 ou 2.

4. Utilisation d'un kit comprenant :
un anticorps qui se lie à la CIRBP, et
au moins un article sélectionné parmi un anticorps isotype témoin relatif audit anticorps, un témoin positif et un témoin négatif, pour déterminer un pronostic de fibrose pulmonaire idiopathique.
